# EUROPEAN PATENT SPECIFICATION

(11) **EP 1 185 224 B1**
(45) Date of publication and mention of the grant of the patent: **07.12.2005**
(21) Application number: 00926592.7
(22) Date of filing: 09.05.2000
(51) Int. Cl.: A61F 13/02

(54) **SKIN ADHESIVE TAPE**
AUF DER HAUT HAFTENDES KLEBEBAND
BANDE ADHESIVE S'APPLIQUANT SUR LA PEAU

(30) Priority: 12.05.1999 WO PCT/BR99/01888
(43) Date of publication of application: 13.03.2002
(73) Proprietor: Johnson & Johnson Industrial Ltda., Sao José dos Campos, SP (BR)
(72) Inventor: DE CARVALHO SCAMILLA ALEDO, Maria, Aparecida, CEP-12242-040 S o José dos Campos, SP (BR); SERRANO, Luiz, Antonio, CEP-08900-000 Guararema, SP (BR); DE OLIVEIRA, D'Artagnan, Silva, CEP-12233-002 S o José dos Campos, SP (BR)
(74) Representative: Mercer, Christopher Paul
(86) International application number: PCT/BR2000/000045
(87) International publication number: WO 2000/069379

(56) References cited:
- WO-A-97/43991
- WO-A-99/16396
- US-A- 2 001 862
- US-A- 3 563 237
- US-A- 3 949 741
- US-A- 4 719 909
- US-A- 4 748 976

## Description

### Field of the Invention

The present invention refers to an adhesive tape to be adhered onto the skin and which may or may not carry absorbent substrates for wounds.

### Background of the Invention

Skin adhesives tapes are known and have been used for a long time for covering and protecting a wound. In a traditional form, it is well known the adhesive tape consisting of a continuous tape coated with a rubber based adhesive and used in the form of rectangular portions which are cut from the continuous tape.

Likewise, the dressings for small wounds are known, which are composed of a substantially rectangular adhesive tape portion, with a central region provided with a small pad of absorbent material.

When it is necessary to apply an adhesive tape to a body region subjected to motion, for instance the hand or other region of complex topography, some inconconveniences and difficulties are met.

For example, when a small wound occurs in a finger, a dressing is typically placed transversaly to the longitudinal imaginary axis of the erect finger, with one of the ends of said dressing overlapping the opposite end, thus surrounding an area corresponding to the surface of a cylinder. As this finger moves, after the application of the dressing, there is a certain discomfort caused by the tightening of the dressing adhered onto itself and very often the adhered surface of the dressing does not resist the tensions generated by the movements and releases, whereby the dressing may easily fall from the finger.

Moreover, if a linear adhesive tape is used to follow formation of wrinkles, folds or corrugations along the structure thereof, bothering the user and establishing a less efficient adhesion. In other words, linear adhesive tapes are poorly adapted to the topography of the body.

There has been a constant search for alternatives turned to the application of adhesives to the human body. Many examples may be mentioned. Some patents present merely decorative forms, lacking functionality, such as USD 377,688 (with the shape of a lightning bolt), USD 392,045 (flower and lace shapes) and USD 340,985 (in the form of a football). Other patents present adhesive bandages for specific regions of the body, such as US 3,971,374 (palm of the hand), USD 295,559 (entire finger), USD 383,544 (finger tips), US 5,792,091 (elbows). US-A-4748976 discloses anatomical dressings for cosmetic treatments of a skin surface, consisting in a sheet of close-textured pure cotton fabric, soaked in or impregnated with a cosmetic preparation held in an oil or grease medium, a portion of which is cut from the sheet to match the contours of that part of the anatomy to be treated and applied thereto, remaining attached to the skin through surface adhesion for a given length of time. Many other forms and arrangements are found, such as US 5,531,999 (wound tape covered with hydrogel), USD 5,538,500 (adhesive bandage with the central core provided with four adherent lateral rods), US 5,000,746 (adherent individual elements connected to each other by a string), USD 393,903 (with a bow tie shape, having cuts to hold tubes or cannulas), USD 364,922 (approximately "i" shaped, in order to hold cannulas, catheters or tubes) and USD 5,820,578 (triangular shape, having cuts on the periphery of the triangle sides, in order to conform to the curves of the body. Neither of these alternatives leads to a skin adhesive tape, which may transmit a feeling of comfort to the user, as a function of a more ergonomic adjustment of the adhesive tape to the user's body, better dissipating the tensions applied to the tape during use or motion and therefore having a lower number of release situations.

US-A-3949741 discloses a method and appliance for reducing facial wrinkles. In the method, a pressure-sensitive adhesive appliance is tightly applied to wrinkle prone areas of the human face in such a manner that the skin under the appliance is placed in a flattened and smoothed configuration and skin cells are stripped from the skin on removal of the appliance. An appliance shaped for removing wrinkles in the infra orbital region below the human eye and extending to the temple past the outside corner of the eye is disclosed.

WO-A-9916396 discloses a dressing for covering a portion of the anatomical surface of a living being, said dressing being able to adhere to the skin, the mucosa and/or a wound on a protruding portion of a living being without exposing the skin to a significant stress after application and said dressing comprising a substantially water-impervious layer or film and a skin-friendly adhesive, which dressing has a low modulus allowing an easy deformation during application but yet a sufficiently high elasticity to essentially prevent formation of wrinkles after application, wherein the dressing is optionally covered in part or fully by one or more release liners or cover films to be removed before or during application and wherein the dressing has the general form of a boomerang.

US-A-3563237 discloses a bandage for a distal phalanx or the like comprising an elongated strip of adhesive coated material, said strip having a pair of longitudinal sides terminating in transverse ends, said longitudinal sides being arcuate and substantially parallel to one another, one of said sides being concave and the other being convex.

WO-A-9743991 discloses a dressing (1) comprising a main part (2) and a handle part, said main part comprising an adhesive layer (13), a release liner (14) and a carrier layer (12) optionally covered by a protective layer (11), wherein the handle part comprises at least one tab member (3,4), said tab member being designed for use as a grip for applying the dressing (1) to the skin without touching the adhesive surface of the main part, and said tab member (3,4) comprising one or more layers (9) and having at least one layer in common with the main part (2) of the dressing (1) characterised in that the length of an intermediate zone between the handle part and the main part is less than 20% of the length of the periphery of the main part. The dressings specifically disclosed are either symmetrical about a longitudinal axis or are generally U-shaped.

US-A-2001862 discloses a tissue support for treating facial lines, comprising a tissue-supporting member adapted to exert opposing forces on opposite sides of a facial line whereby to hold the lined tissue in a stretched condition transversely of said line, an adhesive adapted to secure said member to the facial tissues, the member being formed of a moisture-absorbing material having a capacity for shrinkage upon drying, and a moisture-resisting coating on the outer surface of said member whereby upon loss of moisture incident to drying upon the wearer's face the said member is caused to warp concavely toward the face. The tissue supports specifically disclosed are either generally rectangular or are generally U-shaped.

US-A-4719909 discloses an under-eye light absorbing device and method comprising utilising a light absorbing replaceable sheet material patch attachable by pressure sensitive adhesive to the zygomatic arch skin area adjacent to the lower side of the user's orbit. The devices specifically disclosed are again generally U-shaped.

### Summary of the Invention

Thus, it is the general objective of the present invention to provide a skin adhesive tape, which may be comfortably and securely applied to different regions of the body, transmitting to the user a feeling of higher comfort, as compared to the presently known adhesive tapes.

The skin adhesive tape of the present invention comprises a flexible basic film, which may have one or more layers and whose skin fixation surface incorporates an adhesive coating.

According to the present invention there is provided skin adhesive tape as defined in the appendant claims.

The adhesive tape of the invention comprising a median portion incorporating two end portions, whose longitudinal axes are inclined to opposite sides in relation to the direction defined by the longitudinal axis of the median portion, provides the adhesive tape which may have generally "S" or "Z" shapes.

It is also possible to attach to the median portion an absorbent pad, usually rectangular, to be seated onto the wound to be protected.

The tests made with the adhesive tape defined above have proved that it has been achieved a product which can be more easily adapted to different parts of the body and which may have its basic shape associated with different embodiments for the geometrical arrangement of the end portions, aiming at optimizing its adaptability to determined regions of the body.

### Brief Description of the Drawings

The invention will be described below, with reference to the attached drawings, whose figures are simplified schematic representations of embodiments of the invention, with measurements or proportions that do not necessarily correspond to a real article, since they are intended to clarify the invention.

In the drawings:
Figure 1 illustrates a plan view of an adhesive tape constructed according to the present invention, when observed from its skin fixation surface and incorporating a wound covering pad in the median portion and end portions inclined to opposite sides in relation to the median portion;
Figures 2A, 2B and 2C illustrate, respectively and in a non-exhaustive way, several shapes for the wound covering pad illustrated in figure 1.

### Description of the Illustrated Embodiments

As illustrated in the appended drawings, the skin adhesive tape of the present invention comprises a basic film 10, in any adequate flexible material known by those skilled in the art, particularly PVC by those skilled in the art, particularly PVC (polyvinyl chloride), polyurethane, polyethylene, woven or nonwoven fabrics, which materials may be used individually or combined to each other and said basic film 10 may be continuous or perforated, solid or foamy, or formed by one or two layers.

On the skin fixation surface to be applied to the user's skin, the basic film 10 incorporates a thin adhesive coating 20, which is usually acrylic based and which is also well known in the art as PSA ("pressure sensitive adhesive").

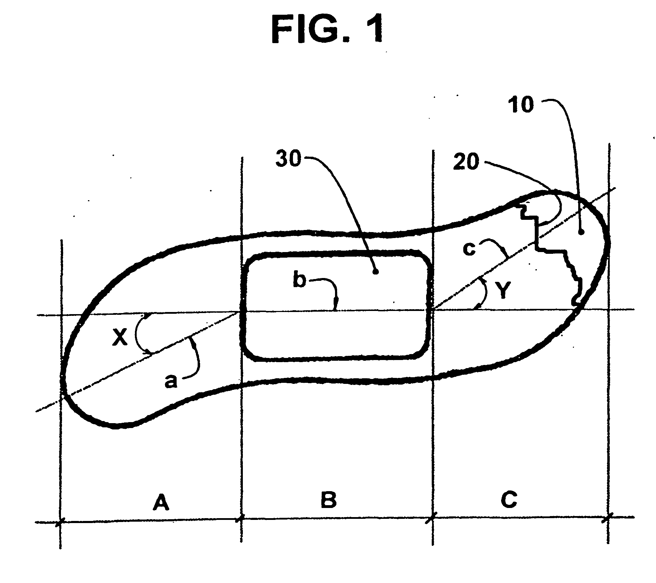

Figure 1 illustrates a skin adhesive tape constructed according to the invention and comprising a basic film 10 in plastic material, with the skin fixation surface incorporating an adhesive coating 20 and, medianly, a pad 30 of adequate material to cover the wound. In the embodiment illustrated in figure 1, the pad 30 has a rectangular contour, with a width inferior to the width of the region of the basic film 10 into which it is incorporated.

As illustrated in figure 1, the skin adhesive tape in question comprises a median portion B, having a longitudinal axis b and incorporating, in a single piece, two opposite end portions A and C, whose longitudinal axes a and c are disposed according to inclined directions in relation to the longitudinal axis of the median portion B.

The median portion B has usually a rectangular shape, the length thereof being, for instance, covered by the pad 30 as illustrated in figure 1. The small width of the pad 30 in relation to the width of the median portion B allows to maintain on the latter two exposed longitudinal lateral strips of the skin fixation surface of the basic film 10 incorporating the adhesive 20.

The pad 30 is made of any material which is adequate to contact the wounds, preferably having absorbent characteristics. In this alternative, the pad 30 is irremovably adhered to the median portion B which serves as a base therefor. The width and length of said pad 30 are preferably smaller than the corresponding dimensions of the median portion B. This pad may have any shape, for example rectangular, circular, semicircular, ellipsoidal, etc. As those skilled in the art know, this pad 30 may be made of any adequate material, such as woven or nonwoven fabric, wood pulp, synthetic fibers, perforated plastic film, etc., and it may contain additives which act on the wounded skin or on the healthy skin around the wounded skin, such as antibiotics, antiseptics and cicatrizants, etc..

The term "pad" further encompasses, for example, a hydrogel or a creamy composition applied onto the skin fixation surface of the median portion B and which is adequate to contact the region of the wounded skin.

In the embodiment of figure 1, the longitudinal axes a and c of the end portions A and C form angles X and Y, which are identical and opposite to the longitudinal axis b of the median portion B, taking into account that in this illustrative construction both end portions A and C are inclined towards opposite sides of the longitudinal axis b of the median portion B, resulting in a shape similar to an "S" or a "Z".

The angles of inclination X, Y of the longitudinal axes a and c of the end portions A and C in relation to the longitudinal axis b of the median portion B usually range from about 5 to about 90 degrees, more preferably from about 10 to about 75 degrees and still more preferably from about 25 to about 60 degrees.

With the constructive arrangement described hereinbefore, the skin adhesive tape in question, when applied for example to the user's finger, assumes approximately a helicoidal shape, surrounding the finger and seating more easily thereon, with less formation of wrinkles and folds than those produced by the traditional adhesive tapes.

As already mentioned, the adhesive tape in question may be shaped in order to have an end portion with the longitudinal axis inclined in relation to the longitudinal axis b of the median portion B. In the embodiments in which both end portions A and C have their longitudinal axes a and c inclined in relation to the longitudinal axis of the median portion b, the angles of inclination X and Y have values which are usually approximately equal.

The skin adhesive tape in question is shaped so as to have the contour of the end portions A and C matching with the contour of the median portion B at the connecting regions with the latter, in order to produce in this place a smooth rounded transition, without forming sharp corners. Besides this constructive aspect, the adhesive tape has its end portions A and C preferably tapered towards the respective ends which are preferably rounded.

As illustrated and described below, the skin adhesive tape of the present invention has a median portion B which, although being generally and substantially rectilinear in its longitudinal extension, may have its lateral edges slightly bent, in order to follow a possible curvature of the end portion A and C, or the change of direction of the latter in relation to the longitudinal axis b of the median portion B.

It should be also understood that both end portions A and C of the adhesive tape may have equal or different lengths in relation to each other, in this last case

Preferably, both end portions A and C have the same length, even if they are subjected to different inclinations in terms of angle and direction. The proportion between the length of the median portion B and the length of any of the end portions A and C ranges preferably between 1:10 and 1:0.5, these lengths being measured along the longitudinal axis of each portion.

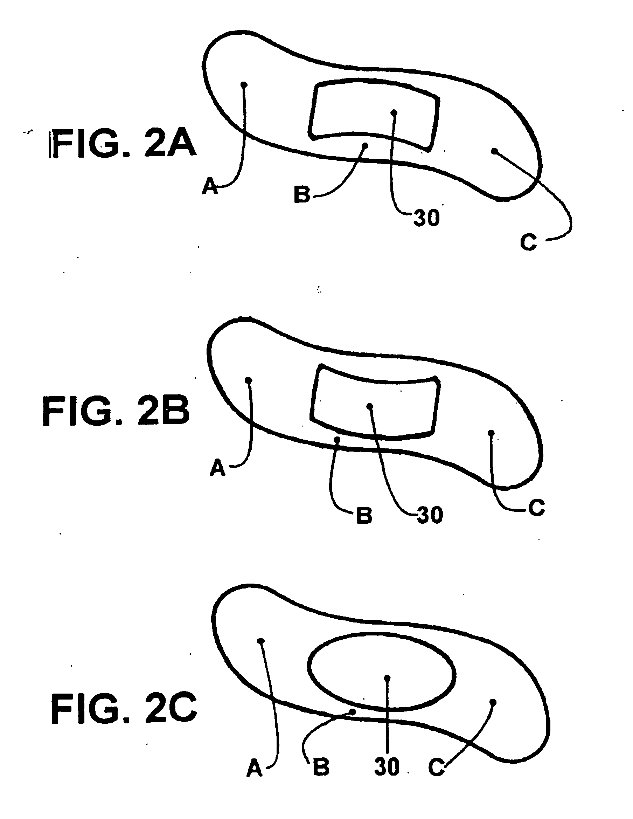

Figures 2A, 2B and 2C illustrate a skin adhesive tape comprising two end portions A and C, which are tapered towards rounded ends inclined to different sides of the longitudinal axis b of the median portion B, into which is incorporated a pad 30, whose contour ranges from the rectangular shape, with bent lateral edges, to the elliptical shape.

## Claims

1. Skin adhesive tape, comprising a flexible basic film (10) in one or more layers and whose skin fixation surface incorporates an adhesive coating (20), and further comprising a median portion (B), having a longitudinal axis (b) and incorporating, in a single piece, only two opposite end portions (A, C), **characterized in that** said end portions (A, C) have their longitudinal axes (a, c) inclined to opposite sides of the longitudinal axis (b).of the median portion (B).

2. Skin adhesive tape, as in claim 1, in which the inclination of the longitudinal axis (a, c) of any of the end portions (A, C) in relation to the longitudinal axis (b) of the median portion (B) ranges from about 5 to about 90 degrees.

3. Skin adhesive tape, as in claim 2, in which the inclination of the longitudinal axis (a, c) of any of the end portions (A, C) in relation to the longitudinal axis (b) of the median portion (B) ranges from about 10 to about 75 degrees.

4. Skin adhesive tape, as in claim 3, in which the inclination of the longitudinal axis (a, c) of any of the end portions (A, C) in relation to the longitudinal axis (b) of the median portion (B) ranges from about 25 to about 60 degrees.

5. Skin adhesive tape, as in claim 1, in which the angles of inclination of the longitudinal axes (a, c) of the end portions (A, C) in relation to the longitudinal axis (b) of the median portion (B) are approximately equal.

6. Skin adhesive tape, as in claim 1, in which at least one of the end portions (A, C) has a rounded end.

7. Skin adhesive tape, as in claim 1, in which the lateral edges of the median portion (B) match with the adjacent lateral edges of the end portions (A, C) in the connecting regions between said portions.

8. Skin adhesive tape, as in claim 1, in which the median portion (B) has its lateral edges slightly bent and matching with the adjacent bent edges of the end portions (A, C).

9. Skin adhesive tape, as in claim 1, in which the proportion between the length of the median portion (B) and the length of any of the end portions (A, C) ranges from 1: 10 to 1: 0.5, being preferably around 1: 1.

10. Skin adhesive tape, as in claim 1, which further comprises a pad (30), of an adequate material to contact wounds and being preferably absorbent, which is incorporated to the skin fixation surface of the median portion (B).

## Patentansprüche

1. Auf der Haut haftendes Klebeband, umfassend einen flexiblen Grundfilm (10) in einer oder mehreren Schichten und dessen Hautfixierungsfläche eine haftende Beschichtung einschließt (20), und weiter umfassend einen Mittelteil (B), der eine längs verlaufende Achse (b) aufweist und in einem Stück lediglich zwei gegenüberliegende Endteile einschließt (A, C), **dadurch gekennzeichnet, daß** die Endteile (A, C) ihre längs verlaufenden Achsen (a, c) schräg zu den gegenüberliegenden Seiten der längs verlaufenden Achse (b) des Mittelteils (B) geneigt aufweisen.

2. Auf der Haut haftendes Klebeband wie in Anspruch 1, wobei die Neigung der längs verlaufenden Achse (a, c) von einem der Endteile (A, C) in Bezug zu der längs verlaufenden Achse (b) des Mittelteils (B) im Bereich von ungefähr 5 bis ungefähr 90 Grad liegt.

3. Auf der Haut haftendes Klebeband wie in Anspruch 2, wobei die Neigung der längs verlaufenden Achse (a, c) von einem der Endteile (A, C) in Bezug zu der längs verlaufenden Achse (b) des Mittelteils (B) im Bereich von ungefähr 10 bis ungefähr 75 Grad liegt.

4. Auf der Haut haftendes Klebeband wie in Anspruch 3, wobei die Neigung der längs verlaufenden Achse (a, c) von einem der Endteile (A, C) in Bezug zu der längs verlaufenden Achse (b) des Mittelteils (B) im Bereich von ungefähr 25 bis ungefähr 60 Grad liegt.

5. Auf der Haut haftendes Klebeband wie in Anspruch 1, wobei die Winkel der Neigung der längs verlaufenden Achsen (a, c) der Endteile (A, C) in Bezug zu der längs verlaufenden Achse (b) des Mittelteils (B) ungefähr gleich sind.

6. Auf der Haut haftendes Klebeband wie in Anspruch 1, wobei wenigstens ein Endteil (A, C) ein abgerundetes Ende aufweist.

7. Auf der Haut haftendes Klebeband wie in Anspruch 1, wobei die lateralen Kanten des Mittelteils (B) mit den angrenzenden lateralen Kanten der Endteile (A, C) in den Verbindungsregionen zwischen den Teilen übereinstimmen.

8. Auf der Haut haftendes Klebeband wie in Anspruch 1, wobei die lateralen Grenzen des Mittelteils (B) leicht gebogen sind und mit den angrenzenden abgerundeten Grenzen der Endteile (A, C) übereinstimmen.

9. Auf der Haut haftendes Klebeband wie in Anspruch 1, wobei das Verhältnis zwischen der Länge des Mittelteils (B) und der Länge von einem der Endteile (A, C) im Bereich von 1:10 bis 1:0,5 liegt, wobei ungefähr 1:1 besonders bevorzugt ist.

10. Auf der Haut haftendes Klebeband wie in Anspruch 1, weiter umfassend ein Kissen (30) aus einem zur Berührung von Wunden adäquaten Stoff, der vorzugsweise absorbierend ist, wobei er in der Hautfixierungsfläche des Mittelteils (B) eingeschlossen ist.

## Revendications

1. Bande adhésive s'appliquant sur la peau, comprenant un film de base flexible (10) en une ou plusieurs couches et dont la surface de fixation à la peau incorpore un revêtement adhésif (20), et comprenant en outre une portion médiane (B) ayant un axe longitudinal (b) et incorporant, en une pièce unique, seulement deux portions d'extrémité opposées (A, C), **caractérisée en ce que** lesdites portions d'extrémité (A, C) ont leurs axes longitudinaux (a, c) inclinés vers les côtés opposés à l'axe longitudinal (b) de la portion médiane (B).

2. Bande adhésive s'appliquant sur la peau, selon la revendication 1, dans laquelle l'inclinaison de l'axe longitudinal (a, c) de l'une quelconque des portions d'extrémité (A, C) est, par rapport à l'axe longitudinal (b) de la portion médiane (B), dans la gamme allant d'environ 5 à environ 90 degrés.

3. Bande adhésive s'appliquant sur la peau, selon la revendication 2, dans laquelle l'inclinaison de l'axe longitudinal (a, c) de l'une quelconque des portions d'extrémité (A, C) est, par rapport à l'axe longitudinal (b) de la portion médiane (B), dans la gamme allant d'environ 10 à environ 75 degrés.

4. Bande adhésive s'appliquant sur la peau, selon la revendication 3, dans laquelle l'inclinaison de l'axe longitudinal (a, c) de l'une quelconque des portions d'extrémité (A, C) est, par rapport à l'axe longitudinal (b) de la portion médiane (B), dans la gamme allant d'environ 25 à environ 60 degrés.

5. Bande adhésive s'appliquant sur la peau, selon la revendication 1, dans laquelle les angles d'inclinaison des axes longitudinaux (a, c) des portions d'extrémité (A, C), par rapport à l'axe longitudinal (b) de la portion médiane (B), sont approximativement égaux.

6. Bande adhésive s'appliquant sur la peau, selon la revendication 1, dans laquelle au moins l'une des portions d'extrémité (A, C) a une extrémité arrondie.

7. Bande adhésive s'appliquant sur la peau, selon la revendication 1, dans laquelle les bords latéraux de la portion médiane (B) correspondent aux bords latéraux adjacents des portions d'extrémité (A, C) dans les régions de liaison entre lesdites portions.

8. Bande adhésive s'appliquant sur la peau, selon la revendication 1, dans laquelle la portion médiane (B) a ses bords latéraux légèrement pliés et. correspondant aux bords pliés adjacents des portions d'extrémité (A, C).

9. Bande adhésive s'appliquant sur la peau, selon la revendication 1, dans laquelle la proportion entre la longueur de la portion médiane (B) et la longueur de l'une quelconque des portions d'extrémité (A, C) est dans la gamme de 1 : 10 à 1 : 0,5, celle-ci étant de préférence d'environ 1 : 1.

10. Bande adhésive s'appliquant sur la peau, selon la revendication 1, qui comprend en outre une compresse (30), d'un matériau adéquat pour être au contact de blessures et étant de préférence absorbant, qui est incorporée à la surface de fixation à la peau de la portion médiane (B).
